# EUROPÄISCHE PATENTANMELDUNG

(11) **EP 3 607 866 A1**
(43) Veröffentlichungstag der Anmeldung: **12.02.2020**
(21) Anmeldenummer: 18188442.0
(22) Anmeldetag: 10.08.2018
(51) Int. Cl.: A47L 25/08, A61L 2/20, C11D 3/00, B65D 81/32

(54) **REINIGUNGSVORRICHTUNG FÜR TEXTILE MATERIALIEN UND REINIGUNGSVERFAHREN**

(71) Anmelder: Stahn, Jens, 11140 Conil De La Frontera (ES)
(72) Erfinder: Stahn, Jens, 11140 Conil De La Frontera (ES)
(74) Vertreter: Von Rohr Patentanwälte Partnerschaft mbB

(57) **Zusammenfassung**

Dargestellt und beschrieben ist eine Reinigungsvorrichtung für textile Materialien, mit wenigstens einem Behälter zur Aufnahme eines ersten flüssigen oder gelartigen Reaktionsmittels. Erfindungsgemäß ist vorgesehen, dass ein zweites flüssiges, gelartiges oder pulverförmiges Reaktionsmittel vorgesehen ist, wobei das erste Reaktionsmittel und das zweite Reaktionsmittel dazu ausgebildet sind, miteinander unter Bildung eines gasförmigen Wirkstoffes zu reagieren.

## Beschreibung

Die Erfindung betrifft eine Reinigungsvorrichtung für textile Materialien, mit wenigstens einem Behälter zur Aufnahme eines ersten flüssigen oder gelartigen Reaktionsmittels. Die Erfindung betrifft ferner ein Reinigungsverfahren insbesondere für textile Materialien.

Die Reinigung von Textilien bzw. textilen Materialien stellt bisweilen eine aufwendige Aufgabe dar. Dies gilt insbesondere, wenn große Mengen Textilien gereinigt werden müssen oder die textilen Materialien nur schwer einer Reinigung zugänglich sind. Letzteres ist üblicherweise bei Textilien im Wohnbereich bzw. bei textilem Mobiliar der Fall. Insbesondere Einrichtungsgegenstände wie Matratzen, Polstermöbel und deren Bezüge, Vorhänge, Teppiche sowie Auslegware sind üblicherweise nicht ohne Weiteres einer Reinigung zuzuführen, sondern müssen an Ort und Stelle behandelt werden. Je nach Zugänglichkeit ist dies mit hohem Aufwand und zum Teil erhöhten Kosten verbunden, wobei oftmals ein nicht zufriedenstellendes Reinigungsergebnis erzielt wird.

Insbesondere in hygienisch kritischen Bereichen, wie beispielsweise Krankenhäusern, Pflegeeinrichtungen und dergleichen, bestehen zudem besondere Anforderungen an die Reinigung. Um ein hygienisch einwandfreies oder in einigen Fällen sogar steriles Ergebnis zu erzielen, ist in der Regel eine extrem aufwendige und entsprechend kostenintensive Reinigung durchzuführen. Gängige Reinigungsmethoden, beispielsweise Absaugen und chemische Behandlung mit Detergenzien, werden hierzu durch weitere Maßnahmen zur Desinfektion, etwa Bestrahlung mit UV-Licht, thermische Behandlung oder Behandlung mit aggressiven Desinfektionsmitteln, ergänzt.

Als weiterer Anwendungsfall besteht darüber hinaus häufig auf Reisen der Wunsch, bestimmte textile Materialien einer gründlichen Reinigung zu unterziehen. Dies schließt beispielsweise textiles Mobiliar mit häufig wechselnder Nutzung mit ein, beispielsweise Betten in Hotels oder Jugendherbergen. In einigen Fällen werden solche Textilien aus Zeit- und Kostengründen nur oberflächlich gereinigt. Im Hinblick auf eine hygienische Sauberkeit in einem zufriedenstellenden Maß kann daher eine zusätzliche Reinigung wünschenswert sein. Spezielle Reinigungsgeräte sind häufig zu groß, um auf Reisen mitgeführt zu werden. Eine Behandlung der betreffenden Oberflächen mit entsprechenden Reinigungsmitteln, etwa Sprays oder Schäumen, geht dagegen mit dem Nachteil einher, dass eine Benutzung unmittelbar nach der Behandlung ausgeschlossen ist. Zuvor muss sich die eingebrachte Feuchtigkeit weitgehend verflüchtigt haben, um das unangenehme Gefühl des Kontakts mit restfeuchten Textilien zu vermeiden.

Ähnliches gilt im Fall von selten genutzten Wohnbereichen, beispielsweise einem Gästezimmer oder in einem Ferienhaus. Nach längerer Lagerung ohne Benutzung kann es je nach Klimabedingungen in Bezug auf Temperatur und Feuchtigkeit zur Bildung von Schimmel oder zum Einnisten von Ungeziefer und Mikroorganismen kommen, verbunden mit unangenehmen Gerüchen. Als weiterer Anwendungsfall kann eine Reinigung benutzter Textilien bei oder nach Krankheit des Benutzers erfolgen, um die Textilien von Keimen und Krankheitserregern zu befreien.

Bekannte Mittel zur Reinigung von textilen Materialien sind in der Regel aufwendig in der Handhabung, erfordern eine hohe Einwirk- und Bearbeitungszeit und sind - je nach zu erzielendem Hygienegrad - teuer in der Anschaffung. Aufgrund ihrer Größe sind entsprechende Vorrichtungen zudem häufig nur eingeschränkt zum Transport, beispielsweise auf Reisen, geeignet. Im Fall gängiger Reinigungssubstanzen zum Sprühen oder Schäumen wird die Zeit bis zur möglichen Wiederbenutzung ferner dadurch verlängert, dass es geraume Zeit in Anspruch nehmen kann, bis sich die eingebrachte Feuchtigkeit verflüchtigt hat. Letzteres Problem tritt insbesondere im Fall von vergleichsweise dicken Materialien, wie Matratzen oder Polstern sowie Kissen, auf.

Vor diesem Hintergrund ist Aufgabe der vorliegenden Erfindung eine Möglichkeit bereitzustellen, auf einfache und kostengünstige Weise textile Materialien zu reinigen und insbesondere von biologischen Verunreinigungen, wie Mikroorganismen oder Krankheitserregern, zu befreien.

Die vorgenannte Aufgabe wird durch eine Reinigungsvorrichtung mit den Merkmalen gemäß dem Anspruch 1 sowie durch ein Verfahren gemäß Anspruch 15 gelöst. Vorteilhafte Weiterbildungen sind jeweils Gegenstand der abhängigen Ansprüche.

Das zugrundeliegende Prinzip der vorliegenden Erfindung ist die Reinigung und insbesondere Desinfektion von textilen Materialien mittels eines gasförmigen Wirkstoffes. Die Behandlung der zu reinigenden Materialien erfolgt daher auf trockene Weise. Die gereinigten Gegenstände oder Oberflächen sind somit unmittelbar nach der Behandlung wieder benutzbar. Zum einen muss eingebrachtes flüssiges Reinigungsmittel nicht zuerst verdampfen. Zum anderen ist die Behandlung mit Flüssigkeiten durch Aufsprühen oder Einschäumen in der Regel mit einer vergleichsweise langen Einwirkzeit verbunden, in der das Reinigungsmittel in das textile Material eindringt. Insbesondere im Fall von größeren textilen Gegenständen, wie Matratzen, Kissen oder Polstern, ist eine entsprechend hohe Zeitspanne abzuwarten, bis ein aufgebrachtes flüssiges Reinigungsmittel für eine Tiefenreinigung ausreichend weit in das Material eingedrungen ist. In gleicher Weise verzögert sich der Zeitpunkt, zu dem das Reinigungsmittel wieder vollständig aus dem Material ausgetreten ist, das Material also durchgetrocknet ist. Neben einem unangenehmen Gefühl bei der Benutzung von textilen Gegenständen mit enthaltener Restfeuchte kann ferner ein unangenehmer Geruch von dem Textilmaterial ausgehen, solange noch gegebenenfalls aggressive Reinigungsmittel aus seinem Inneren Austreten.

Die erfindungsgemäße Vorrichtung sieht die Erzeugung eines gasförmigen Wirkstoffs vor, der durch zwei miteinander reagierende Substanzen erzeugt wird. Das Gas durchdringt aufgrund seiner hohen Beweglichkeit die Faserstruktur der zu reinigenden Textilmaterial und wirkt in kurzer Zeit auch in innenliegenden Schichten. Die Ausbreitung des entstehenden gasförmigen Wirkstoffs aufgrund von Diffusionsvorgängen ermöglicht es, auch schwer zugängliche Stellen in ausreichendem Maß zu erreichen, um eine gründliche Reinigung bzw. Desinfektion zu gewährleisten. In der weiteren Folge nimmt die Konzentrationsdichte des Wirkstoffs infolge der fortschreitenden Diffusion sukzessive ab. Somit verflüchtigt sich der Wirkstoff letztlich selbstständig aus dem textilen Material. Es muss hierbei insbesondere kein Phasenübergang des Wirkstoffs, d.h. beispielsweise dessen Verdampfung, stattfinden, um das textile Gewebe von dem Wirkstoff zu befreien. Insbesondere bleibt keine Restfeuchtigkeit zurück.

Dadurch, dass der gasförmige Wirkstoff durch eine Reaktion zweier Reaktionsmittel erst kurz vor seiner Anwendung erzeugt wird, lässt sich die erfindungsgemäße Vorrichtung problemlos lagern und transportieren bis sie eingesetzt wird. Zur Inbetriebnahme werden ein erstes flüssiges oder gelartiges Reaktionsmittel und ein zweites flüssiges, gelartiges oder pulverförmiges Reaktionsmittel derart miteinander vermischt, dass eine Reaktion der beiden Substanzen unter Bildung eines gasförmigen Wirkstoffes erfolgt.

Die beiden Reaktionsmittel können in einem Gehäuse separat voneinander gelagert werden. Bei der Aktivierung erfolgt die Vermischung der beiden Substanzen dann insbesondere im Innern des Gehäuses, dass vom gasförmigen Wirkstoff als Reaktionsprodukt verlassen. Der Wirkstoff verteilt sich daraufhin in der Umgebung der Vorrichtung und gerät in Kontakt mit dort positionierten Gegenständen, insbesondere textilen Materialien.

Nach der Verwendung kann das Gehäuse der Vorrichtung bevorzugt wiederverwendet werden. Hierzu werden die verwendeten Verbrauchsmaterialien entfernt und durch neue ersetzt. Zu diesem Zweck können entsprechende Behälter mit den Reaktionsmitteln vorgehalten werden, mit welchen die Vorrichtung neu bestückt wird.

Als besonders geeigneter Wirkstoff hat sich in Versuchen Chlordioxid herausgestellt. Als gasförmige Substanz mit einer stark desinfizierenden Wirkung durchdringt Chlordioxid ohne Weiteres die zu reinigenden textilen Materialien und befreit diese wirksam von Keimen, Viren, Sporen, Bakterien, sowie Parasiten und unangenehmen Gerüchen, die durch mikrobiologische Prozesse entstehen. Darüber hinaus wirkt Chlordioxid auch fungizid, so dass ebenfalls eine Schimmelbekämpfung mit der erfindungsgemäßen Vorrichtung auf einfache Weise möglich ist. Chlordioxid lässt sich zudem auf unkomplizierte Weise aus der Reaktion von vergleichsweise kostengünstigen Substanzen gewinnen.

Nach dem Vermischen zweier entsprechender Komponenten für die Reaktion zur Erzeugung von Chlordioxid entsteht dieses in geringer Dosis, die jedoch aufgrund der hohen Wirksamkeit von Chlordioxid als Desinfektionsmittel für die Reinigung üblicher Mengen textiler Materialien ausreicht. Chlordioxid ist ferner als Molekül instabil und zerfällt nach einiger Zeit in ungefährliche Substanzen. Als Reaktionspartner zur Erzeugung von Chlordioxid eignet sich insbesondere Natriumchlorit, das bei Kontakt mit einer Säure, beispielsweise Salzsäure, Milchsäure oder Essigsäure, unter Bildung von Chlordioxidgas reagiert. Dementsprechend handelt es sich bei einem der Reaktionsmittel vorzugsweise um Natriumchlorit oder eines der Reaktionsmittel enthält Natriumchlorit. Bei dem jeweils anderen Reaktionsmittel kann es sich letztlich um eine beliebige Säure handeln. Natriumchlorit ist einfach verfügbar und im Gegensatz zu Chlordioxid lager- und transportstabil. Als weiterer Vorteil lässt sich Natriumchlorit durch eine nahezu beliebige Säure aktivieren. Bevorzugt kommt eine 25%ige Natriumchlorit-Lösung als eines der Reaktionsmittel zum Einsatz.

Das entstehende Chlordioxid zerfällt aufgrund seiner Instabilität nach und nach und verliert dadurch seine giftige Wirkung. Eine übliche Halbwertszeit beim Abbau des Chlordioxids beträgt etwa 30 Minuten bei Raumtemperatur. In wärmeren Umgebungen kann ein deutlich schnellerer Zerfall auftreten. Umgekehrt führen niedrigere Temperaturen üblicherweise zu einer Verlängerung der Halbwertszeit. Letztlich zerfällt das Chlordioxid selbstständig und hinterlässt dabei im wesentlichen keine gesundheitsschädlichen Rückstände.

Werden Reaktionsmittel ausgewählt, die unter Reaktion miteinander einen Wirkstoff mit einer hohen Wirksamkeit generieren oder die zu einer hohen Ausbeute an Wirkstoff führen, reicht eine vergleichsweise geringe Menge der Reaktionsmittel für typische Anwendungen der erfindungsgemäßen Reinigungsvorrichtung aus. Insbesondere kann die jeweils vorgehaltene Menge des ersten und/oder des zweiten Reaktionsmittels kleiner oder gleich 10 ml, vorzugsweise kleiner oder gleich 5 ml, bevorzugt kleiner oder gleich 3 ml, betragen. Dies reduziert zum einen die Kosten zur Herstellung der erfindungsgemäßen Vorrichtung. Zum anderen lässt sich die Vorrichtung hierdurch besonders kompakt ausbilden und somit beispielsweise auf Reisen mitführen. Ferner wird durch eine geringere Menge enthaltener flüssiger oder gelartiger Reaktionsmittel das Risiko von Verunreinigungen oder gegebenenfalls körperlicher Irritationen entsprechend vermindert.

Je nach Anwendungszweck kann die Reinigungsvorrichtung in verschiedenen Größen ausgebildet werden. Zur Reinigung kleinerer Möbel o.ä. reicht insbesondere eine Vorrichtung entsprechend geringer Baugröße aus. Eine kompaktere Bauform ist auch bevorzugt, wenn die Transportabilität der Vorrichtung im Vordergrund steht. Zur Reinigung größerer Bereiche, beispielsweise ganzer Bodenbeläge, kann die erfindungsgemäße Reinigungsvorrichtung entsprechend größer, d.h. zur Bildung einer größeren Menge des Wirkstoffs, ausgebildet werden.

In einer bevorzugten Ausführungsform der erfindungsgemäßen Vorrichtung sind zwei separate Behälter vorgesehen, die jeweils zur Aufnahme von Reaktionsmitteln vorgesehen sind bzw. Reaktionsmittel enthalten. Bei den Behältern kann es sich um wiederverwendbare bzw. neu befüllbare Behältnisse oder Fächer im Gehäuse der Vorrichtung handeln. Alternativ oder zusätzlich können auch mit den Reaktionsmitteln gefüllte Fläschchen, Beutel und/oder Schlauchabschnitte als Behälter im erfindungsgemäßen Sinne fungieren. Derartige separat vorgesehene Behälter besitzen den Vorteil, auf einfache Weise, insbesondere nach einmaliger Verwendung, entsorgt und durch neue, befüllte Behälter ersetzt werden zu können, so dass die Vorrichtung rasch für einen erneuten Einsatz vorbereitet ist. Die erfindungsgemäße Reinigungsvorrichtung lässt sich im Ergebnis sauber befüllen und transportieren, da die Behälter erst am Anwendungsort geöffnet und die enthaltenen Reaktionsmittel miteinander zur Reaktion vermischt werden.

Eine besonders sichere und zuverlässige Handhabung der erfindungsgemäßen Reinigungsvorrichtung wird durch eine separate Reaktionsstruktur in einem Reaktionsbereich des Gehäuses, beispielsweise einer entsprechenden Kammer, erreicht. Die Reaktionsstruktur ist dazu ausgebildet, die flüssigen, gelartigen oder pulverförmigen Reaktionsmittel zumindest weitgehend aufzunehmen und diese am Austreten zu hindern. Dabei soll jedoch eine ausreichende Vermischung der beiden Komponenten innerhalb der Reaktionsstruktur möglich bleiben. Durch eine vollständige Aufnahme der flüssigen Substanzen wird verhindert, dass diese aus dem Gehäuse der Vorrichtung ungewünscht austreten, dort zu Verunreinigungen führen können und dann insbesondere nicht für die beabsichtigte Reaktion zur Erzeugung des Wirkstoffs zur Verfügung stehen.

Die erfindungsgemäße Vorrichtung kann in ihrem inneren insbesondere drei Bereiche oder Fächer aufweisen, in denen die zwei Reaktionsmittel, gegebenenfalls in entsprechenden Behältern, und die Reaktionsstruktur vorgesehen sind. Zur Aktivierung der Vorrichtung werden die Reaktionsmittel aus ihren Bereichen in den Bereich mit der Reaktionsstruktur eingebracht und dort von dieser aufgenommen. Die Vermischung der Substanzen und das damit verbundene Einsetzen der Reaktion zur Generierung des gasförmigen Wirkstoffs setzt daraufhin im Innern der Reaktionsstruktur ein.

Die Reaktionsstruktur ist zumindest bereichsweise bevorzugt als insbesondere saugfähiges Gewebe und/oder porös ausgebildet, so dass eine ausreichende Aufnahmekapazität für flüssige und/oder gelartige Substanzen gewährleistet ist. Die Reaktionsstruktur kann diesbezüglich eine schwammartige bzw. offenporige Struktur aufweisen, die ein besonders leichtes Eindringen von flüssigen Substanzen in das Innere ermöglicht. Das Eindringen der Reaktionsmittel kann insbesondere durch eine kapillare Wirkung unterstützt werden.

In einer alternativen Ausführungsform der erfindungsgemäßen Vorrichtung ist das zweite Reaktionsmittel im benutzungsfertigen Zustand der Vorrichtung bereits als Flüssigkeit, Gel oder Pulver in die Reaktionsstruktur eingebettet. In diesem Fall ist lediglich ein Fach oder ein Behälter mit einem weiteren Reaktionsmittel erforderlich, dass in die entsprechend präparierte Reaktionsstruktur eingebracht wird. Daraufhin setzt die Reaktion der beiden Komponenten, d.h. des eingebetteten Reaktionspartners und des von außen eingebrachten Mittels, ein. Durch die von vornherein gegebene Bindung des zweiten Reaktionsmittels in der Reaktionsstruktur wird die Handhabung der erfindungsgemäßen Vorrichtung zum einen weiter vereinfacht und zum anderen in der Handhabungssicherheit erhöht. Da letztlich nur eine Substanz auf eine entsprechende Struktur aufgebracht bzw. in die Struktur eingebracht werden muss, verringert sich das Risiko von Fehlern in der Handhabung. Insbesondere ist ein Verschütten bzw. ein versehentliches Austreten von flüssigen Substanzen beim Vermischen der zwei Reaktionspartner in diesem Fall nicht möglich. Die Reaktionsstruktur kann mit dem zweiten Reaktionsmittel getränkt sein, falls dieses in flüssiger Form vorliegt. Eine Einbettung des Reaktionsmittels ist jedoch auch in Gelform oder in Form eines Pulvers möglich.

Das Gehäuse der erfindungsgemäßen Reinigungsvorrichtung weist vorzugsweise Öffnungen an einer Mehrzahl von Seiten, bevorzugt an allen Seiten, auf. Durch mehrere Öffnungen bzw. einer Anordnung von Öffnungen rund um die Vorrichtung wird das Ausströmen des gasförmigen Wirkstoffes bei der Reaktion der Reaktionsmittel erleichtert. Die Reinigungsvorrichtung kann somit mittig in einem zu reinigenden Bereich, beispielsweise auf einer Matratze, in einem Bett, auf einem Sofa oder auf einem Teppich, platziert werden. Der ausströmende Wirkstoff erreicht von einer zentralen Stelle aus gleichermaßen alle Bereiche des zu reinigenden textilen Materials.

Der Ablauf der Reaktion und somit die Bereitstellung des Wirkstoffs lässt sich durch eine optionale Heizeinrichtung zur Erwärmung des ersten Reaktionsmittels und/oder des zweiten Reaktionsmittels beschleunigen. Die Erwärmung kann hierbei sowohl vor als auch während der Reaktion erfolgen. Als Heizeinrichtung eignen sich neben elektrischen Heizungen beispielsweise auch Systeme, die aufgrund einer exothermen chemischen Reaktion Wärme bereitstellen oder Latentwärmespeicher, welche die in ihnen enthaltene Wärme nach einer Aktivierung an die Umgebung freisetzen. Durch eine Heizeinrichtung lässt sich die erfindungsgemäße Vorrichtung zudem auch in kalten Umgebungen, beispielsweise in unbeheizten Wohnbereichen, einsetzen. Dies kann etwa dann wünschenswert sein, wenn ein Ferienhaus oder eine Hütte zur winterlichen Benutzung nach der Ankunft zunächst gereinigt werden muss, falls sich aufgrund einer längeren Nichtbenutzung Verunreinigungen in Form von Schimmel oder unangenehme Gerüche gebildet haben. Die wirksame Anwendung der erfindungsgemäßen Reinigungsvorrichtung kann somit erst nicht nach dem Aufheizen des Raumes in dem betreffenden Bereich erfolgen, sondern kann unmittelbar nach der Ankunft in Gang gesetzt werden. Hieraus ergibt sich eine erhebliche Zeitersparnis für den Benutzer, der den gereinigten Wohnraum bzw. die gereinigten textilen Elemente mit geringerer Verzögerung in vollem Umfang nutzen kann.

Die erfindungsgemäße Reinigungsvorrichtung kann ferner eine Ventilationseinrichtung aufweisen, mittels derer der gasförmige Wirkstoff nach seinem Entstehen bewegt werden kann. Hierdurch wird die Verteilung des Wirkstoffes in der Umgebung der Vorrichtung begünstigt und gegebenenfalls das Ausströmen des Wirkstoffes gefördert. Eine schnellere Verteilung des Wirkstoffes in der Umgebung führt zu einem entsprechend schnellen Wirkungseintritt und einem raschen Durchdringen textiler Strukturen. Wird beispielsweise Chlordioxid als Wirkstoff zur Desinfektion verwendet, setzt der molekulare Zerfall bereits unmittelbar nach der Entstehung ein und schreitet zunehmend fort. Durch eine beschleunigte Verteilung des Wirkstoffs kann somit die wirksame Ausbeute erhöht werden. D.h. eine im Vergleich größere Zahl nicht zerfallener und damit wirksamer Moleküle wird in die zu reinigende Bereiche gebracht. Im Vergleich hierzu zerfällt bei einer rein diffusionsbasierten Verteilung des gasförmigen Wirkstoffs ein nennenswerter Teil der Moleküle bereits bevor der zu reinigende Gegenstand erreicht wird. Eine entsprechende Ventilationseinrichtung kann elektrisch betrieben, beispielsweise in Form eines Propellerlüfters, ausgebildet sein. Alternativ oder zusätzlich kann ferner durch eine bestimmte Geometrie des Gehäuses der erfindungsgemäßen Vorrichtung eine passive Be- bzw. Entlüftung und/oder eine Durchströmung des Innenraums erreicht werden, beispielsweise nach dem Bernoulli-Prinzip.

Wenngleich die Reinigungswirkung des entstehenden gasförmigen Wirkstoffs in der Regel aufgrund mikrobiologischer Vorgänge entstandene unangenehme Gerüche im Textil beseitigt, lässt sich durch Beigabe eines Duftstoffes zum ersten und/oder zweiten Reaktionsmittel ein angenehmer Duft bei der Anwendung der Reinigungsvorrichtung verbreiten. Ein solcher Duft kann zum einen zu einer ansprechenden Raumatmosphäre beitragen und somit die Wirkung eines Duftspenders bzw. Lufterfrischers in der erfindungsgemäßen Reinigungsvorrichtung vereinen. Zum anderen kann durch die Beaufschlagung des gereinigten textilen Materials mit einem Duftstoff das erfolgreiche Reinigen bzw. Desinfizieren des Materials angezeigt und später nachvollzogen werden. Alternativ lässt sich durch Beigabe eines als unangenehm empfundenen Duftstoffes auch eine warnende Wirkung hervorrufen. Dies ist insbesondere in solchen Fällen sinnvoll, wenn als Wirkstoff eine Substanz zum Einsatz kommt, die im aktiven, d.h. nicht zerfallenen Stadium giftig, reizend und/oder auf sonstige Weise gesundheitsschädlich ist, dabei jedoch keinen oder nur einen geringen Eigengeruch aufweist.

Die erfindungsgemäße Vorrichtung weist in einer besonders bevorzugten Ausgestaltung wenigstens einen Abstandshalter auf, der verhindert, dass Ausströmöffnungen im Gehäuse durch anliegende Materialien verschlossen werden. In einem solchen Fall würde das Ausströmen des Wirkstoffes unterdrückt, so dass er ungenutzt im Innern der Vorrichtung verbleiben würde, bis ggf. die Moleküle des Wirkstoffes zerfallen sind. Ein entsprechender Abstandshalter erlaubt das Ausströmen aus dem Innern der Vorrichtung in ihre Umgebung, von wo aus der Wirkstoff sich diffusiv weiter ausbreiten kann. Durch eine Anordnung von Abstandshaltern an Stellen, die von einem Verlegen durch äußere Materialien betroffen sein können, lässt sich das gewünschte Ausströmverhalten des Wirkstoffes gewährleisten. Ein Abstandshalter ist dementsprechend insbesondere oberseitig vorgesehen. Ferner kann die Vorrichtung auch einen oder mehrere Füße aufweisen, die letztlich als unterseitige Abstandshalter fungieren und ein Ausströmen des Wirkstoffs auch über die Unterseite der Vorrichtung erlauben.

Eine vorzeitige Verflüchtigung des gasförmigen Wirkstoffes wird durch eine Folie verhindert, mittels derer die erfindungsgemäße Reinigungsvorrichtung und das zu reinigende Material umhüllt werden können. Hierdurch wird der Wirkungsbereich des Wirkstoffes auf die zu reinigenden Materialien begrenzt und die Konzentration des Wirkstoffs in einem für die Reinigung bzw. Desinfektion geeigneten Maß aufrechterhalten. Eine Umhüllung in Form einer Folie erlaubt zudem eine Verwendung einer vergleichsweise geringen Menge von Reaktionsmitteln mit der Folge einer entsprechend geringen Menge des erzeugten Wirkstoffs. Durch die Begrenzung des Volumens, in dem sich der Wirkstoff ausbreiten kann, muss letztlich nur eine ausreichende Menge Wirkstoff erzeugt werden, um das Volumen auszufüllen. Insbesondere muss keine derart große Menge des Wirkstoffs erzeugt werden, dass trotz der freien Verflüchtigung im Raum eine ausreichende Konzentration im Bereich der zu reinigenden Materialien bereitgestellt wird. Ferner verhindert die Umhüllung ein Austreten von aggressivem Wirkstoff während dessen aktiver Phase. Während der erfindungsgemäßen Reinigung wird somit durch die Folie auch der Kontakt des Benutzers mit dem Wirkstoff verhindert. Nach Abschluss der Reinigung, d.h. insbesondere nach dem Zerfall des Wirkstoffs, kann der Benutzer die Folie entfernen. Es treten ihm daraufhin gegebenenfalls nur gesundheitlich unbedenkliche Zerfallsprodukte entgegen.

In Versuchen hat sich gezeigt, dass der durch die Folie umschlossene Raum ausreichend groß bemessen ist, wenn er ein Volumen von höchstens 5 m³, vorzugsweise höchstens 3 m³, bevorzugt höchstens 2 m³, besonders bevorzugt höchstens 1 m³, aufweist. Hierdurch wird eine kompakte Baugröße der erfindungsgemäßen Vorrichtung und der Folie gewährleistet. Es ist in diesem Zusammenhang insbesondere vorteilhaft, wenn die Folie während der Anwendung nicht allzu stark von den zu reinigenden textilen Materialien beabstandet ist. Hierdurch wird ein großes ungenutztes Volumen vermieden, welches vom Wirkstoff zwar gefüllt würde, jedoch ohne dass dieser seine Wirkung entfalten könnte. Ein kleineres Volumen konzentriert den Wirkstoff in höherem Maße auf und begünstigt ein tieferes Eindringen in das textile Material. Dies ist insbesondere im Fall von großvolumigen textilen Gegenständen von Vorteil.

Zur gezielten Beeinflussung des Strömungs- und Verteilungsverhaltens des gasförmigen Wirkstoffes kann die Folie ferner gasundurchlässig und gasdurchlässige Bereiche aufweisen. Sollte es erforderlich sein, kann hierdurch die Konzentration des Wirkstoffs auf ein optimales Maß begrenzt gehalten werden. Etwaig überschüssiger Wirkstoff wird in diesem Fall über gasdurchlässige Bereiche der Folie in die äußere Umgebung abgegeben. In den überwiegenden Anwendungsfällen, insbesondere bei einer Verwendung von Chlordioxid als Wirkstoff, ist jedoch eine vollständig gasdichte Folie zur Umhüllung der Wirkbereiche bevorzugt. Im Hinblick auf eine umweltgerechte Entsorgung und geringe Herstellungskosten der Folie ist diese bevorzugt aus einem recycelten und/oder recycelfähigen Material hergestellt oder weist ein solches auf. Alternativ oder zusätzlich kann die Folie auch aus einem biologisch abbaubaren Material bestehen oder ein solches aufweisen.

Bei einer weiteren bevorzugten Ausführungsform der Reinigungsvorrichtung ist eine Auslöseeinrichtung vorgesehen, mittels derer die Reinigungsvorrichtung aktiviert werden kann. Dies geschieht dadurch, dass ein Inkontakttreten der beiden Reaktionsmittel durch Betätigung der Auslöseeinrichtung bewirkt wird. Die Auslöseeinrichtung kann beispielsweise als eine Art Dorn, als Schlägel o.ä. ausgebildet sein und ggf. einen Druckknopf, Hebel, Schalter oder dergleichen umfassen. Letztlich wird durch die Auslöseeinrichtung wenigstens ein Behälter geöffnet, der mit einem ersten Reaktionsmittel gefüllt ist, das daraufhin freigegeben wird. Je nach Ausgestaltung der Reinigungsvorrichtung, d.h. mit zwei separaten Behältern für die Reaktionsmittel oder mit einem in eine Reaktionsstruktur eingebetteten zweiten Reaktionsmittel, werden gegebenenfalls auch mehrere Behältnisse mit Reaktionsmitteln nach der Betätigung der Auslöseeinrichtung eröffnet. Letztlich wird durch die Freigabe des oder der Reaktionsmittel deren Inkontakttreten und Vermischen, insbesondere in der Reaktionsstruktur, bewirkt. Die Reaktion zur Erzeugung des gasförmigen Wirkstoffes tritt üblicherweise von selbst nach dem gegenseitigen Kontakt der Reaktionsmittel auf. Zur Aktivierung der Reinigungsvorrichtung genügt somit die Betätigung der Auslöseeinrichtung.

Die erfindungsgemäße Reinigungsvorrichtung weist vorzugsweise einen Bereich auf, in welchem die Folie für den Transport verstaut werden kann. Dort lässt sich die Folie insbesondere flach gefaltet unterbringen und zur Benutzung entnehmen oder herausziehen. Die Reinigungsvorrichtung lässt sich somit zusammen mit der Folie in kompakter Form auf einfache Weise transportieren.

Die Verwendung der Reinigungsvorrichtung erfolgt typischerweise folgendermaßen: die Reinigungsvorrichtung enthält zwei Reaktionsmittel, die in unvermischter Form im inneren bereitgehalten werden. In diesem Ausgangszustand wird die Reinigungsvorrichtung in die Nähe ihres Einsatzortes verbracht. Dort wird sie geöffnet. Daraufhin werden einer oder mehrere Behälter im Innern, welche die Reaktionsmittel enthalten, geöffnet, beispielsweise aufgebrochen, aufgestochen oder aufgeschnitten. Hierdurch wird jeweils das enthaltene Reaktionsmittel freigegeben und dringt in einen Reaktionsbereich, gegebenenfalls mit einer Reaktionsstruktur zur Aufnahme der Reaktionsmittel, vor, wo sich die Reaktionsmittel vermischen. Hierdurch wird eine Reaktion in Gang gesetzt, durch welche ein gasförmiger Wirkstoff, beispielsweise Chlordioxid, entsteht.

Nach dem Freisetzen und Vermischen der Reaktionsmittel wird die Reinigungsvorrichtung geschlossen und an ihren endgültigen Einsatzort gestellt oder gelegt. Insbesondere durch Öffnungen im Gehäuse der Vorrichtung strömt nun der gasförmige Wirkstoff als Ergebnis der in Gang gesetzten Reaktion der beiden Reaktionsmittel aus und verteilt sich in der Umgebung der Vorrichtung. Dort gerät der Wirkstoff mit zu reinigenden textilen Materialien in Kontakt und entfaltet seine reinigende und/oder desinfizierende Wirkung. Gegebenenfalls wird nach dem Platzieren der Reinigungsvorrichtung am Einsatzort mit einer optionalen Folie eine Umhüllung der Vorrichtung sowie der zu behandelnden Bereiche vorgenommen.

In diesem Status wird die Vorrichtung für einige Zeit nach Möglichkeit unangetastet belassen. Eine gängige Einwirkzeit beträgt beispielsweise 3 Stunden. Nach dieser Zeit kann davon ausgegangen werden, dass die Wirkung des gasförmigen Wirkstoffes vollständig oder zumindest in ausreichendem Maß erfolgt ist. Sofern ein Wirkstoff eingesetzt wird, der von selbst in unbedenkliche Bestandteile zerfällt, beispielsweise Chlordioxid, wird eine Zeit abgewartet, nach deren Verstreichen davon ausgegangen werden kann, dass der überwiegende Teil des aktiven Wirkstoffs abgebaut wurde.

Nach der im Einzelfall unterschiedlichen und bedarfsweise zu bemessenden Einwirkzeit wird die Reinigungsvorrichtung, gegebenenfalls zuvor auch die Folie, von ihrem Anwendungsort entfernt, geöffnet und die enthaltenen Substanzen, die aus der erfolgten Reaktion als nicht flüchtige Bestandteile im Innern der Vorrichtung zurückgeblieben sind, werden entfernt. Gegebenenfalls wird ferner auch die Reaktionsstruktur entfernt.

Die Reinigungsvorrichtung kann daraufhin erneut mit Reaktionsmitteln und gegebenenfalls einer Reaktionsstruktur und/oder einer Folie ausgestattet werden, so dass sie für den nächsten Einsatz vorbereitet ist.

Nachfolgend wird die vorliegende Erfindung anhand bevorzugter Ausführungsbeispiele näher erläutert. Dabei bilden alle beschriebenen und/oder bildlich dargestellten Merkmale jeweils eigenständige Aspekte der Erfindung, unabhängig von ihrer Kombination in den Ausführungsbeispielen oder den Ansprüchen.

Es zeigt
Fig. 1 eine perspektivisch schematische Darstellung einer bevorzugten Ausführungsform der erfindungsgemäßen Reinigungsvorrichtung,
Fig. 2 eine schematische Darstellung des inneren Aufbaus einer bevorzugten Ausführungsform der erfindungsgemäßen Reinigungsvorrichtung in Draufsicht,
Fig. 3 eine schematische Darstellung einer weiteren bevorzugten Ausgestaltung der erfindungsgemäßen Reinigungsvorrichtung in Draufsicht,
Fig. 4 eine schematische Darstellung einer typischen Anwendungssituation für die erfindungsgemäße Reinigungsvorrichtung und
Fig. 5 eine schematische Querschnittsdarstellung einer alternativen Ausführungsform der erfindungsgemäßen Reinigungsvorrichtung in seitlicher Ansicht.

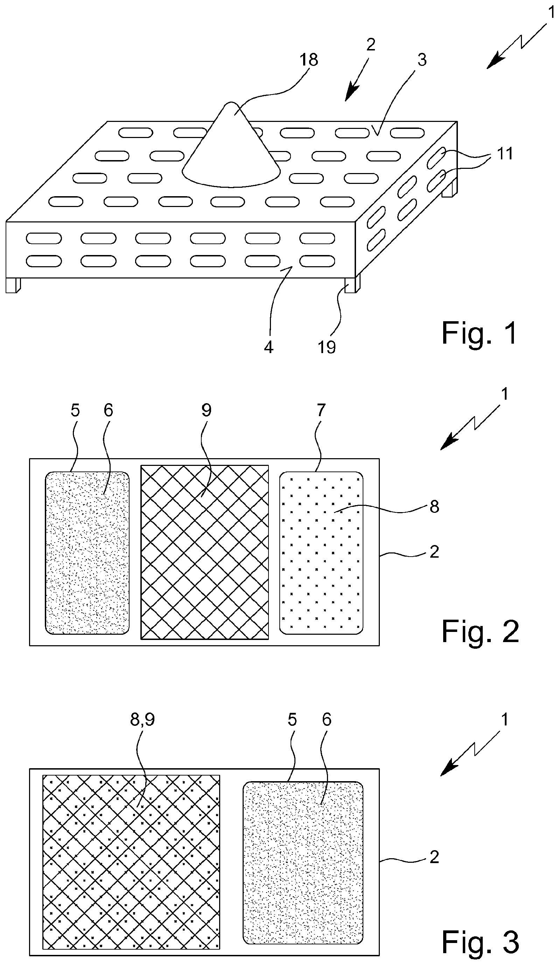

Fig. 1 zeigt eine erfindungsgemäße Reinigungsvorrichtung 1 in einer bevorzugten Ausführungsform. Die Reinigungsvorrichtung 1 umfasst ein Gehäuse 2 mit einer Oberseite 3 sowie Seitenwänden 4.

Der Innenraum der Reinigungsvorrichtung 1 ist durch Öffnen des Gehäuses 2 zugänglich. Hierzu kann die Oberseite 3 des Gehäuses 2 abnehmbar und/oder öffenbar, beispielsweise durch Aufschwenken oder Aufschieben, ausgebildet sein. Alternativ können die Oberseite 3 und die Seitenwände 4 gemeinsam einen insbesondere abnehmbaren Deckel bilden.

Der innere Aufbau der Reinigungsvorrichtung 1 ist beispielhaft in den Fig. 2 und 3 in Draufsicht dargestellt. Fig. 2 zeigt eine Ausführungsform der Reinigungsvorrichtung 1, bei der ein erster Behälter 5 mit einem ersten Reaktionsmittel 6 und ein zweiter Behälter 7 mit einem zweiten Reaktionsmittel 8 separat vorgesehen sind. Die Behälter 5, 7 sind vorliegend als separate Kunststoffsäckchen ausgebildet, können jedoch auch in Form von Glasampullen o.ä. vorliegen.

Die Behälter 5, 7 mit den Reaktionsmitteln 6, 8 sind im vorliegenden Beispiel voneinander beabstandet angeordnet, wobei sich zwischen den Behältern 5, 7 ein Bereich ergibt, in welchem die Reaktionsmittel 6, 8 miteinander reagieren können. In diesem Bereich zwischen den Behältern 5, 7 weist die Reinigungsvorrichtung 1 im vorliegenden Beispiel eine Reaktionsstruktur 9 auf, die zur vollständigen Aufnahme der Reaktionsmittel 6, 8 dient.

Die Reaktionsstruktur 9 besteht aus einem porösen, saugfähigen Material, das zwar eine vollständige oder zumindest weitgehende Aufnahme beider Reaktionsmittel 6, 8 erlaubt, jedoch eine ausreichende Vermischung der beiden Substanzen in der Reaktionsstruktur 9 gewährleistet.

Aufgrund der Reaktion der beiden Reaktionsmittel 6, 8 entsteht ein gasförmiger Wirkstoff 10, der von der Reaktionsstruktur 9 ausgehend den Innenraum der Reinigungsvorrichtung 1 füllt und durch Öffnungen 11 im Gehäuse 2 entweicht.

Die Öffnungen 11 sind im gezeigten Ausführungsbeispiel rund um das Gehäuse 2 angeordnet. D.h. es sind sowohl in allen Seitenwänden 4 als auch an der Oberseite 3 und darüber hinaus auch an der Unterseite des Gehäuses 2 Öffnungen 11 vorgesehen, wobei letztere nicht im Einzelnen zeichnerisch dargestellt sind.

Fig. 3 zeigt eine alternative Ausgestaltung der Reinigungsvorrichtung 1, bei welcher ein erster Behälter 5 mit dem ersten Reaktionsmittel 6 vorgesehen ist, das zweite Reaktionsmittel 8 jedoch bereits in die Reaktionsstruktur 9 eingebettet ist. In diesem Fall reicht zur Aktivierung die Öffnung des ersten Behälters 5, um das erste Reaktionsmittel 6 in die Reaktionsstruktur 9 eindringen zu lassen, wo es mit dem zweiten Reaktionsmittel 8 unter Bildung des gasförmigen Wirkstoffs 10 reagiert.

In den hier vorgestellten Beispielen handelt es sich bei dem ersten Reaktionsmittel 6 um eine beliebige Säure, beispielsweise um Essigsäure. Als zweites Reaktionsmittel 8 kommt vorliegend Natriumchlorit zum Einsatz. Bei einer Vermischung reagieren diese beiden Substanzen unter Bildung von gasförmigem Chlordioxid miteinander, das eine desinfizierende Wirkung besitzt und somit eine Reinigung textiler Materialien, mit denen es in Kontakt kommt, bewirkt. Zusätzlich kann zumindest ein Reaktionsmittel 6, 8 einen Duftstoff enthalten.

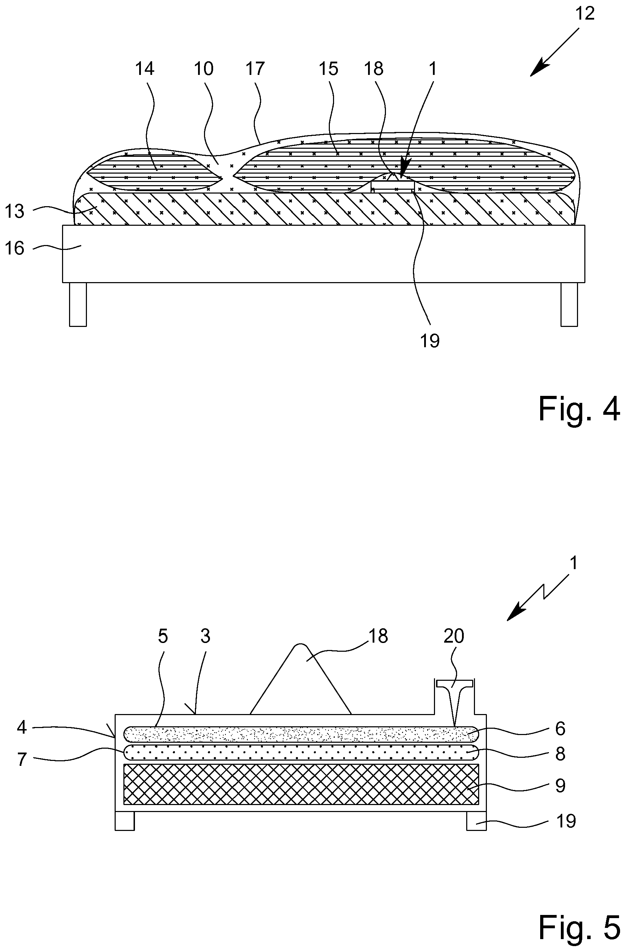

Eine typische Anwendungssituation der erfindungsgemäßen Reinigungsvorrichtung 1 ist in Fig. 4 gezeigt. Bei dem zu reinigenden Gegenstand handelt es sich im dargestellten Beispiel um ein Bett 12. Die großvolumigen textilen Komponenten des Bettes 12, die in der Regel nur schwer einer gründlichen Reinigung unterzogen werden können, lassen sich nach dem erfindungsgemäßen Prinzip leicht und bis in die Tiefe reinigen und desinfizieren.

Konkret wird die Reinigung einer Matratze 13, eines Kissens 14 und einer Bettdecke 15 durchgeführt. Die genannten Komponenten können zum einen gemeinsam gereinigt werden und zum anderen während der Reinigung an Ort und Stelle, d.h. auf dem Gestell 16 des Bettes 12, verbleiben.

Zur Reinigung wird die Reinigungsvorrichtung 1 mit den enthaltenen Reaktionsmitteln 6, 8 in die Nähe der zu reinigenden Materialien gebracht. Vorliegend wird hierzu die Reinigungsvorrichtung 1 etwa mittig auf die Matratze 13 gestellt. Nach Öffnung der Behälter 5, 7 im Innern der Reinigungsvorrichtung 1 treten die Reaktionsmittel 6, 8 in Kontakt miteinander, so dass die gewünschte Reaktion eintritt, bei welcher der gasförmige Wirkstoff 10 entsteht und sich im Gewebe der umliegenden Textilien verteilt.

In der dargestellten bevorzugten Ausführungsform ist zusätzlich eine Folie 17 vorgesehen, mittels derer die zu reinigenden Teile des Bettes 12 umhüllt werden. Durch die Folie 17 bleibt der Wirkstoff 10 in dem Bereich eingeschlossen, in dem er seine Reinigungswirkung entfalten soll.

Die Folie 17 ist aus einem recyclingfähigen Material gebildet und kann nach der Anwendung problemlos der Wiederverwertung zugeführt werden.

Die Folie 17 kann zum einfacheren Transport im Innern der Reinigungsvorrichtung 1 oder an dieser befestigt aufbewahrt werden bis sie zur Anwendung kommt. Hierzu kann die Reinigungsvorrichtung 1 ein separates Fach oder eine entsprechende Tasche aufweisen. Alternativ kann die Folie 17 auch zusammen mit den Behältern 6, 8 und ggf. der Reaktionsstruktur 9 im Innern des Gehäuses 2 der Reinigungsvorrichtung 1 aufbewahrt werden.

Die Folie 17 umschließt vorliegend ein Volumen, das nicht über ein Maß von 1 m³ hinausgeht. Dies ist ausreichend, um die Matratze 13, sowie das Kissen 14 und die Bettdecke 15 vollständig zu umhüllen.

Üblicherweise ist die Menge der Reaktionsmittel 6, 8 so bemessen, dass bei der Reaktion eine vergleichsweise geringe Menge des Wirkstoffs 10 entsteht. Beispielsweise sind in den meisten Fällen 3 ml eines Reaktionsmittels 6, 8 ausreichend, um genügend Wirkstoff entstehen zu lassen, dass etwa ein Bett 12 problemlos vollständig gereinigt bzw. desinfiziert werden kann.

Die Reinigungsvorrichtung 1 weist im dargestellten Beispiel einen oberseitigen Abstandshalter 18 auf. Der Abstandshalter 18 verhindert, dass die Öffnungen 11 im Gehäuse 2 durch anliegende Materialien, beispielsweise die Folie 17 oder die zu reinigenden Textilien verdeckt werden, wie am Beispiel der Bettdecke 15 in Fig. 4 gezeigt ist.

Ferner weist die Reinigungsvorrichtung 1 unterseitige Füße 19 auf, denen eine ähnliche Funktion wie dem Abstandshalter 18 zukommt. Durch die Füße 19 wird eine Beabstandung des Gehäuses 2 von einer Oberfläche bewirkt, auf welcher die Reinigungsvorrichtung 1 abgestellt ist. Im Beispiel der Fig. 4 wird konkret ein Abstand zwischen der Reinigungsvorrichtung 1 und der Matratze 13 erreicht. Hierdurch kann der Wirkstoff 10 nach unten aus dem Gehäuse 2 ausströmen und sich zusätzlich in seitlicher Richtung verteilen, anstatt unmittelbar in die Matratze 13 einzudringen. Die diffusive Verteilung des Wirkstoffs 10 im Innern der Matratze 13 erfolgt erheblich langsamer als im Bereich oberhalb der Matratze 13. Indem ein seitliches Ausströmen erlaubt wird, kann der Wirkstoff 10 somit rasch über einen vergleichsweise großen Bereich in die Matratze 13 eindringen.

Der Darstellung von Fig. 5 ist eine alternative Ausführungsform der erfindungsgemäßen Reinigungsvorrichtung 1 zu entnehmen, bei der eine Auslöseeinrichtung 20 vorgesehen ist. Mittels der Auslöseeinrichtung 20 kann die Reinigungsvorrichtung 1 auf sehr einfache Weise aktiviert werden. Ein Öffnen des Gehäuses 2 und ein einzelnes bzw. manuelles Öffnen der Behälter 5, 7 zur Freigabe der Reaktionsmittel 6, 8 ist dabei insbesondere nicht erforderlich.

Die Auslöseeinrichtung 20 kann, wie abgebildet, als Druckknopf mit einem gekoppelten Dorn ausgebildet sein, der die entsprechend angeordneten Behälter 5, 7 durchsticht, wenn der Druckknopf betätigt wird. Nach der Betätigung ergießen sich die Reaktionsmittel 6, 8 in die Reaktionsstruktur 9 oder allgemein in einen Reaktionsbereich, beispielsweise eine Reaktionskammer. Dort findet die Vermischung der Substanzen statt, so dass in der einsetzenden Reaktion der gasförmige Wirkstoff 10 freigesetzt wird.

Wenngleich diese nicht im Einzelnen zeichnerisch dargestellt ist, kann erfindungsgemäß zusätzlich oder alternativ zu den gezeigten Merkmalen eine Heizeinrichtung vorgesehen sein, mittels derer sich die Temperatur im Bereich der ablaufenden Reaktion und/oder die Temperatur eines oder mehrerer Reaktionsmittel erhöhen lässt. Hierdurch kann der Reaktionsablauf beschleunigt oder - in kalten Umgebungen - das Einsetzen der Reaktion überhaupt erst ermöglicht werden.

Durch eine optionale Ventilationseinrichtung lässt sich das Ausströmen und/oder die Verteilung des Wirkstoffs 10 unterstützen bzw. beschleunigen. Somit werden verlässlich auch zu reinigende Bereiche vom Wirkstoff 10 erreicht, die weiter von der Reinigungsvorrichtung 1 entfernt sind, wenn diese an einer bestimmten Stelle positioniert wurde.

### Bezugszeichenliste:

- 1: Reinigungsvorrichtung
- 2: Gehäuse
- 3: Oberseite
- 4: Seitenwand
- 5: erster Behälter
- 6: erstes Reaktionsmittel
- 7: zweiter Behälter
- 8: zweites Reaktionsmittel
- 9: Reaktionsstruktur
- 10: Wirkstoff
- 11: Öffnung
- 12: Bett
- 13: Matratze
- 14: Kissen
- 15: Bettdecke
- 16: Gestell
- 17: Folie
- 18: Abstandshalter
- 19: Fuß
- 20: Auslöseeinrichtung

## Patentansprüche

1. Reinigungsvorrichtung (1) für textile Materialien, mit wenigstens einem Behälter (5) zur Aufnahme eines ersten flüssigen oder gelartigen Reaktionsmittels (6) **dadurch gekennzeichnet,**
**dass** ein zweites flüssiges, gelartiges oder pulverförmiges Reaktionsmittel (8) vorgesehen ist, wobei das erste Reaktionsmittel (6) und das zweite Reaktionsmittel (8) dazu ausgebildet sind, miteinander unter Bildung eines gasförmigen Wirkstoffes (10) zu reagieren.

2. Reinigungsvorrichtung nach Anspruch 1, **dadurch gekennzeichnet, dass** der Wirkstoff (10) Chlordioxid ist oder Chlordioxid aufweist.

3. Reinigungsvorrichtung nach Anspruch 1 oder 2, **dadurch gekennzeichnet, dass** eine Reaktionsstruktur (9) zur Aufnahme der Reaktionsmittel (6, 8) vorgesehen ist, wobei, vorzugsweise, die Reaktionsstruktur (9) zumindest bereichsweise porös, insbesondere schwammartig, ausgebildet ist.

4. Reinigungsvorrichtung nach einem der vorhergehenden Ansprüche, **dadurch gekennzeichnet, dass** die enthaltende Menge des ersten Reaktionsmittels (6) und/oder des zweiten Reaktionsmittels (8) höchstens 10 ml, vorzugsweise höchstens 5 ml, bevorzugt höchstens 3 ml, beträgt.

5. Reinigungsvorrichtung nach einem der vorhergehenden Ansprüche, **dadurch gekennzeichnet, dass** zwei separate Behälter (5, 7) mit Reaktionsmitteln (6, 8) und/oder zur Aufnahme von Reaktionsmitteln (6, 8) vorgesehen sind.

6. Reinigungsvorrichtung nach einem der vorhergehenden Ansprüche, **dadurch gekennzeichnet, dass** das zweite Reaktionsmittel (8) in die Reaktionsstruktur (9) eingebettet ist.

7. Reinigungsvorrichtung nach einem der vorhergehenden Ansprüche, **dadurch gekennzeichnet, dass** wenigstens ein Reaktionsmittel (6, 8) Natriumchlorit ist oder Natriumchlorit enthält.

8. Reinigungsvorrichtung nach einem der vorhergehenden Ansprüche, **dadurch gekennzeichnet, dass** wenigstens ein insbesondere oberseitiger Abstandshalter (18) vorgesehen ist.

9. Reinigungsvorrichtung nach einem der vorhergehenden Ansprüche, **dadurch gekennzeichnet, dass** eine Folie (17) zur Umhüllung der Reinigungsvorrichtung (1) und des zu reinigenden Materials vorgesehen ist, wobei die Folie zumindest bereichsweise gasundurchlässig ausgebildet ist.

10. Reinigungsvorrichtung nach Anspruch 9, **dadurch gekennzeichnet, dass** der durch die Folie (17) umschlossene Raum ein Volumen von höchstens 5 m³, vorzugsweise höchstens 3 m³, bevorzugt höchstens 2 m³, besonders bevorzugt höchstens 1 m³ aufweist.

11. Reinigungsvorrichtung nach einem der vorhergehenden Ansprüche, **dadurch gekennzeichnet, dass** eine Heizeinrichtung zur Erwärmung des ersten Reaktionsmittels (6) und/oder des zweiten Reaktionsmittels (8) vorgesehen ist.

12. Reinigungsvorrichtung nach einem der vorhergehenden Ansprüche, **dadurch gekennzeichnet, dass** eine Ventilationseinrichtung zum Bewegen des gasförmigen Wirkstoffes (10) vorgesehen ist.

13. Reinigungsvorrichtung nach einem der vorhergehenden Ansprüche, **dadurch gekennzeichnet, dass** das erste Reaktionsmittel (6) und/oder das zweite Reaktionsmittel (8) einen Duftstoff enthält.

14. Reinigungsvorrichtung nach einem der vorhergehenden Ansprüche, **dadurch gekennzeichnet, dass** eine Auslöseeinrichtung (20) vorgesehen ist, die dazu ausgebildet ist, ein Inkontakttreten der Reaktionsmittel (6, 8) zu bewirken.

15. Reinigungsverfahren insbesondere für textile Materialien, wobei eine Reinigungsvorrichtung (1) nach einem der vorhergehenden Ansprüche in der Umgebung eines zu reinigenden Materials angeordnet wird und die Vorrichtung durch Vermischung zweier Reaktionsmittel (6, 8) aktiviert wird, wobei ein gasförmiger Wirkstoff (10) in die Umgebung abgegeben wird.
